(19) Europäisches Patentamt
European Patent Office
Office européen des brevets

(11) Publication number: **0 059 987**
**B1**

(12) # EUROPEAN PATENT SPECIFICATION

(45) Date of publication of patent specification: **14.08.85**

(51) Int. Cl.[4]: **C 07 C 133/12** // C07D259/00

(21) Application number: **82102293.6**

(22) Date of filing: **30.05.80**

(60) Publication number of the earlier application in accordance with Art. 76 EPC: **0 021 121**

(54) **Alpha-cyano-alpha-((N-guanidino)-imino) toluenes.**

(30) Priority: **01.06.79 GB 7919257**

(43) Date of publication of application: **15.09.82 Bulletin 82/37**

(45) Publication of the grant of the patent: **14.08.85 Bulletin 85/33**

(84) Designated Contracting States: **BE CH DE FR GB LI LU NL SE**

(56) References cited:
**AT-B- 190 941**
**CH-A- 511 216**
**DE-A-1 802 364**
**GB-A-1 223 491**
**GB-A-1 318 645**
**US-A-2 952 677**

(73) Proprietor: **THE WELLCOME FOUNDATION LIMITED**
**183-193 Euston Road**
**London NW1 2BP (GB)**

(72) Inventor: **Baxter, Martin George**
**34 Whitehead Close**
**Wilmington Dartford Kent (GB)**
Inventor: **Elphick, Albert Reginald**
**51 Baring Road**
**Lee London, S.E. 12 (GB)**
Inventor: **Miller, Alistair Ainslie**
**91 Elmshurst Gardens**
**Tombridge Kent (GB)**
Inventor: **Sawyer, David Alan**
**60 Bourne Vale**
**Hayes Kent (GB)**

(74) Representative: **Berg, Wilhelm, Dr. et al**
**Patentanwälte Dr. Berg Dipl.-Ing. Stapf Dipl.-Ing. Schwabe Dr. Dr. Sandmair Postfach 86 02 45**
**Stuntzstrasse 16**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 059 987 B1

Courier Press, Leamington Spa, England.

**Description**

The present invention relates to a group of novel compounds useful for the preparation of specific substituted aromatic compounds, which are the subject matter of the European parent patent application No. 80 103 032.1 and which are useful in the treatment of CNS disorders, such as epilepsy.

The present application therefore relates to the compound of the formula (I)

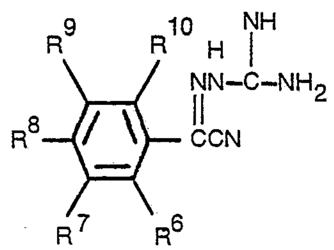

(I)

wherein $R^6$ is chlorine, bromine, iodine, $C_{1-4}$ alkyl or trifluoromethyl; $R^7$ is hydrogen, halogen, $C_{1-4}$ alkyl or trifluoromethyl or $R^6$ and $R^7$ form a —CH=CH—CH=CH— group optionally substituted by a halogen atom or a $C_{1-4}$ alkyl or trifluoromethyl group; $R^8$ is hydrogen, halogen, $C_{1-4}$ alkyl or trifluoromethyl; $R^9$ is hydrogen, halogen, $C_{1-4}$ alkyl or trifluoromethyl, and $R^{10}$ is hydrogen, methyl or fluorine,
provided that at most only two of $R^7$—$R^{10}$ are other than hydrogen and that $R^7$—$R^{10}$ are not all hydrogen when $R^6$ is chlorine.

Suitably the $C_{1-4}$ alkyl group is a methyl group. Suitably $R^6$ is a chlorine or bromine atom or a methyl or trifluoromethyl group or is linked to $R^7$ to form a —CH=CH—CH=CH— group and preferably $R^6$ is a chlorine or bromine atom or linked to $R^7$ to form a —CH=CH—CH=CH— group.

Preferably $R^7$ and $R^9$ are each hydrogen, chlorine or bromine atoms. Preferably $R^8$ is a hydrogen or bromine atom.

When three of the substituents $R^6$—$R^{10}$ are other than hydrogen, it is preferred that $R^8$ and $R^{10}$ are hydrogen and $R^6$, $R^7$ and $R^9$ are those halogen atoms previously defined and in particular chlorine atoms.

The present compounds of the above noted formula (I) can be used for the preparation of compounds of formula (II):

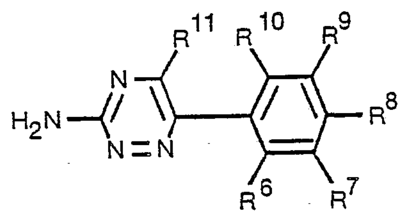

(II)

wherein $R^6$, $R^7$, $R^8$, $R^9$ and $R^{10}$ are as defined above and $R^{11}$ is amino, $C_{1-4}$ acylamino or di-substituted aminomethyleneamino. Said compounds are obtained by the cyclisation of a compound of the above noted formula I and thereafter, where desired, substituting the amino group adjacent to the phenyl ring to give a group $R^{11}$, wherein $R^{11}$ is as herein before defined other than amino, by conventional methods. This cyclisation reaction is normally carried out by refluxing in an alkanol, preferably a $C_{14}$ alkanol such as methanol or ethanol, in the presence of a strong base such as potassium hydroxide.

The compounds of the formula (II), being the subject matter of the European parent application No. 80 102 032.1 and which are obtainable via the present compounds of the formula (I) are active in the treatment of CNS disorders, such as psychiatric and neurological disorders and are particularly useful as anticonvulsants, for example in the treatment of epilepsy. Furthermore these triazines are believed to be non-depressant at likely therapeutic dose levels and therefore are advantageous as compared with depressant antiepileptics such as phenobarbitone.

The preparation of the present compounds of the formula (I) is analogous to that described in the literature, i.e. US-Patent No. 3 637 688, for structurally related compounds.

The following Examples illustrate the preparation of the compounds of the invention and the preparation of formula (II) obtainable therewith.

Example 1

Preparation of α-Cyano-α-[(N-guanidino)-imino]-2,3-dichloro-toluene of the formula

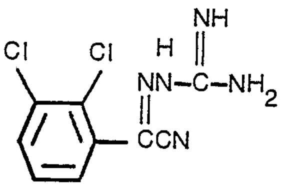

*2,3-Dichlorobenzoic Acid*

A solution of 2,3-dichloroiodobenzene (37.3 g, 0.14 M) in sodium dried ether (300 mls) was added dropwise to magnesium turnings (3.65 g, 0.15 gm Atm) and a crystal of iodine with warming so as to form a Grignard reagent.

The mixture was stirred and refluxed for 2 hours then cooled and transferred dropwise, under nitrogen, into a stirred mixture of sodium dried ether (250 mls) containing solid carbon dioxide (*ca.* 100 g). The mixture was stirred for 2 hours, left overnight to warm to room temperature, then treated with ice (*ca.* 150 g) and 2N aqueous hydrochloric acid (75 mls), and the product extracted with ether (200, 100 and 50 mls). The combined ether extracts were washed with water (2 × 40 mls) then repeatedly extracted with 2N aqueous sodium hydroxide (100, 50 and 50 mls). These basic solutions were combined, stirred with activated charcoal (3 g) for 10 minutes, filtered and the cooled filtrate was acidified with concentrated hydrochloric acid (25 mls) at 10°C. The resultant solid was filtered off, washed with water (2 × 20 mls) and dried *in vacuo*. Yield 20.76 g (77.6%), m.p. 167—169°C (uncorrected).

*2,3-Dichlorobenzoyl Chloride*

A mixture of 2,3-dichlorobenzoic acid (39.4 g 0.2 M) and thionyl chloride (100 mls) was heated to reflux for 2½ hours. The cooled solution was evaporated down *in vacuo* and distilled under nitrogen. Yield 35.5 g (85%), b.p. 146—148°C at 31 mm of mercury pressure.

*2,3-Dichlorobenzoyl Cyanide*

A mixture of cuprous cyanide (36.9 g, 0.41 M), potassium iodide (68.5 g, 0.41 M) and xylene (400 mls) was refluxed in an atmosphere of nitrogen under a Dean and Stark trap for 24 hours so as to remove all trace of water.

A solution of 2,3-dichlorobenzoyl chloride (35.5 g, 0.17 M) in sodium dried xylene (130 mls) was added dropwise to the above mixture of dry cuprous cyanide and xylene. The resulting mixture was stirred and heated to reflux for a further 72 hours. The cooled mixture was filtered and the solid washed well with sodium dried xylene (200 mls). The filtrate and washings were combined and evaporated down *in vacuo* to give an oil. Yield 32 g (94%).

*α-Cyano-α-[(N-guanidino)-imino]-2,3-dichloro-toluene*

A solution of 2,3-dichlorobenzoyl cyanide (32 g, 0.16 M) in dimethylsulphoxide (80 mls) was added dropwise to a stirred suspension of aminoguanidine bicarbonate (81.67 g, 0.6 M) which had been treated with 8N aqueous nitric acid (400 mls) at a temperature of *ca* 25°C. The mixture was stirred for 3 hours, then left to stand at room temperature for 7 days. The cooled mixture was stirred and basified with 0.880 aqueous ammonia (400 mls) at 20°C, then stirred with ice cooling for 30 minutes, filtered and the resulting solid washed thoroughly with water and finally dried *in vacuo*. Thereby the title compound is obtained as a solid.

Adding above solid to a 10% solution of potassium hydroxide pellets in methanol (400 mls), heating the solution to reflux for 1½ hours, cooling the solution, evaporating down *in vacuo,* treating with ice water (800 mls), stirring for 30 minutes and filtering gives a residue, which when dried and recrystallised from isopropanol gives 3,5-diamino-6-(2,3-dichlorophenyl)-1,2,4-triazine. Yield 6.8 g (15.6%), m.p. 216—218°C (uncorrected).

Example 2

Preparation of α-Cyano-α-[(N-guanidino)-imino]-2,3,5-trichloro-toluene

*2,3,5-Trichlorobenzoic Acid*

Powered sodium nitrite (37.0 g, 0.54 M) was added portionwise to concentrated sulphuric acid (270 ml) which was stirred under an atmosphere of nitrogen. The temperature of the mixture was not allowed to rise above 70°. Meanwhile 3-amino-2,5-dichlorobenzoic acid (100 g, 0.45 M) was dissolved in hot glacial acetic acid (1,200 ml), the resultant solution was cooled rapidly to room temperature and added dropwise to the above stirred and cooled nitrous acid mixture so that the internal temperature did not rise above 30°. The solution formed after the addition was left at room temperature for 2 hours then was slowly added to a stirred solution of cuprous chloride (97 g, 0.97 M) in concentrated hydrochloric acid (970 ml). The resultant

mixture was stirred until the nitrogen evolution had ceased and was then left overnight. The solid was filtered off, washed well with water and dried *in vacuo*. Yield 90.1 g (89%) m.p. 164—165°C (uncorrected).

*2,3,5-Trichlorobenzoyl Chloride*

A mixture of 2,3,5-trichlorobenzoic acid (90 g, 0.4 M) and dimethyl formamide (1 ml) in thionyl chloride (200 ml) was heated to reflux for 2 hours. The cooled solution was evaporated down *in vacuo* and the residue distilled under nitrogen. Yield 89.2 g (90%), b.p. 158—160°C at the pressure of 30 mm of mercury.

*2,3,5-Trichlorobenzoyl Cyanide*

A mixture of cuprous cyanide (89 g, 0.9 M), potassium iodide (150.5 g, 0.9 M) and xylene (800 ml) was heated to reflux in an atmosphere of nitrogen under a Dean and Stark trap for 24 hours.

A solution of 2,3,5-trichlorobenzoyl chloride (89 g, 0.36 M) in sodium dried xylene (100 ml) was added to the above suspension. The resulting mixture was stirred and heated to reflux for a further 72 hours. The cooled mixture was filtered and the solid was washed well with sodium dried xylene (200 ml). The filtrate and washings were combined and evaporated *in vacuo* to give an oil. Yield 76 g (96%).

*α-Cyano-α-[(N-guanidino)-imino]-2,3,5-trichloro-toluene*

A solution of 2,3,5-trichlorobenzoyl cyanide (38.5 g, 0.16 M) in dimethylsulphoxide (80 ml) was added dropwise to a stirred suspension of aminoguanidine bicarbonate (65.76 g, 0.32 M) which had been treated with 8N aqueous nitric acid (560 ml). The mixture was stirred for 3 hours and then was left to stand at room temperature for 21 days. The solid was filtered off, washed with water (2 × 100 ml) and dried *in vacuo*. Thereby the title compound is obtained as a solid.

Heating a suspension of the dried solid in a 10% solution of potassium hydroxide pellets in methanol (320 ml) to reflux for 1 hour, cooling the mixture and evaporating down *in vacuo*, treating the residue with ice/water (200 ml), filtering off the resultant solid, drying *in vacuo*, putting this dried solid on top of a dry column (25 mm diameter, 200 g of MFC silica gel), eluting with a solution of ethyl acetate/methanol/acetic acid (90:2.5:2.5), collecting fractions 50 to 80 (900 drops per fraction), combining and evaporating down *in vacuo*, leaves a resultant solid which when crystallised from isopropanol gives 3,5-diamino-6-(2,3,5-trichorophenyl)-1,2,4-triazine. Yield 0.77 g (1.6%), m.p. 232—235°C (uncorrected).

**Claim**

A compound of the formula (I)

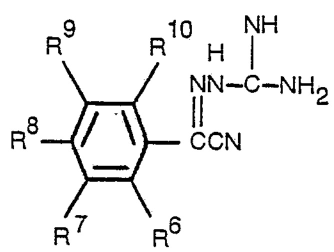

(I)

wherein $R^6$ is chlorine, bromine, iodine, $C_{1-4}$ alkyl or trifluoromethyl; $R^7$ is hydrogen, halogen, $C_{1-4}$ alkyl or trifluoromethyl or $R^6$ and $R^7$ form a —CH=CH—CH=CH— group optionally substituted by a halogen atom, a $C_{1-4}$ alkyl or trifluoromethyl group; $R^8$ is hydrogen, halogen, $C_{1-4}$ alkyl or trifluoromethyl; $R^9$ is hydrogen, halogen, $C_{1-4}$ alkyl or trifluoromethyl and $R^{10}$ is hydrogen, methyl or fluorine,
provided that at most only two or $R^7$—$R^{10}$ are other than hydrogen and that $R^7$—$R^{10}$ are not all hydrogen when $R^6$ is chlorine.

**Patentanspruch**

Verbindung der allgemeinen Formel I

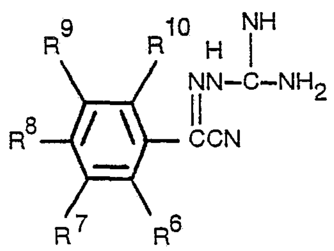

(I)

worin $R^6$ für Chlor, Brom, Jod, $C_{1-4}$-Alkyl oder Trifluormethyl steht, $R^7$ ein Wasserstoffatom, ein Halogen-

atom oder einen $C_{1-4}$-Alkyl oder Trifluormethylrest darstellt oder $R^6$ und $R^7$ einen Rest —CH=CH—CH=CH— bilden, der gegebenenfalls durch ein Halogenatom, einen $C_{1-4}$-Alkylrest oder einen Trifluormethylrest substituiert ist, $R^8$ Wasserstoff, Halogen, $C_{1-4}$-Alkyl oder Trifluormethyl ist, $R^9$ für Wasserstoff, Halogen, $C_{1-4}$-Alkyl oder Trifluormethyl steht und $R^{10}$ Wasserstoff, Methyl oder Fluor ist, mit der Maßgabe, daß höchstens nur zwei der Reste $R^7$—$R^{10}$ andere Reste als Wasserstoff sind und die Reste $R^7$—$R^{10}$ nicht alle Wasserstoffatome sind, wenn $R^6$ ein Chloratom ist.

**Revendication**

Composé de formule (I)

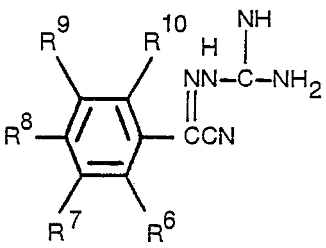

(I)

où $R^6$ est un atome de chlore, de brome ou d'iode ou radical alcoyle en $C_{1-4}$ ou trifluorométhyle; $R^7$ est un atome d'hydrogène ou d'halogène ou radical alcoyle en $C_{1-4}$ ou trifluorométhyle ou bien $R^6$ et $R^7$ forment un radical —CH=CH—CH=CH— éventuellement substitué par un atome d'halogène ou un radical alcoyle en $C_{1-4}$ ou trifluorométhyle; $R^8$ est un atome d'hydrogène ou d'halogène ou radical alcoyle en $C_{1-4}$ ou trifluorométhyle; $R^9$ est un atome d'hydrogène ou d'halogène ou radical alcoyle en $C_{1-4}$ ou trifluorométhyle et $R^{10}$ est un atome d'hydrogène ou de fluor ou radical méthyle,
avec la restriction qu'au maximum deux d'entre $R^7$—$R^{10}$ sont autres que des atomes d'hydrogène et que $R^7$—$R^{10}$ ne sont pas tous des atomes d'hydrogène lorsque $R^6$ est un atome de chlore.